# EUROPEAN PATENT APPLICATION

(11) **EP 4 202 061 A1**
(43) Date of publication of application: **28.06.2023**
(21) Application number: 21858652.7
(22) Date of filing: 20.08.2021
(51) Int. Cl.: C12Q 1/6883, C12Q 1/6869

(54) **BIOMARKER FOR DIAGNOSING AGE-RELATED MACULAR DEGENERATION, AND USE THEREOF**

(30) Priority: 20.08.2020 KR 20200104923
(71) Applicant: Genome Opinion Inc., Seoul 04799 (KR); Seoul National University Hospital, Seoul 03080 (KR)
(72) Inventor: SUN, Choong Hyun, Seoul 04799 (KR); KIM, Su Gyeong, Seoul 04799 (KR); IM, Ho Gune, Seoul 04799 (KR); SONG, Han, Seoul 04799 (KR); OH, Baek Lok, Seoul 07004 (KR); CHOI, Su-Yeon, Seoul 05504 (KR)
(74) Representative: Dehns
(86) International application number: PCT/KR2021/011149
(87) International publication number: WO 2022/039565

(57) **Abstract**

The present invention relates to a marker composition, a kit, a gene panel, and a method which are for providing information for predicting the occurrence of, diagnosing, or treating age-related macular degeneration. The present invention is a novel tool that can provide information for predicting the occurrence of, diagnosing, or treating age-related macular degeneration, and has excellent sensitivity and can be easily analyzed without the use of a biopsy, and thus can be effectively used for the early diagnosis of age-related macular degeneration.

## Description

### Technical Field

The present invention relates to a biomarker capable of predicting occurrence of or diagnosing age-related macular degeneration and uses thereof.

### Background Art

Macular degeneration is an eye disease in which degeneration occurs in the macular area and causes visual impairment. At the beginning of the disease, the field of vision is blurred and the near vision is distorted, which later leads to blindness. The main cause of macular degeneration is aging, followed by genetic factors, and environmental factors include ultraviolet rays, smoking, high-fat, high-calorie westernized diet, and the like.

Age-related macular degeneration (AMD) causes severe and irreversible vision loss and is known as the leading cause of blindness in the population over 50 years of age. Age-related macular degeneration can be divided into two types: dry-type (atrophic) and wet-type (exudative). For the dry-type, waste products are accumulated in the macular area, and vision changes may occur in the early stages; however, it is simply known as a symptom due to aging. In addition, the wet-type is a severely advanced form of macular degeneration, in which abnormal blood vessels grow under the macula and retina so that exudate or blood leaks out, the macular is damaged and healthy cells are destroyed, which may eventually lead to vision loss.

Generally, for the age-related macular degeneration, once vision impairment begins, there are many cases in which previous vision cannot be restored. Thus, early detection thereof is very important. The early detection can be achieved through regular ophthalmologic examinations by an ophthalmologist. If age-related macular degeneration is suspected through ophthalmic examination including fundus examination, definite diagnosis can be made by performing in-depth ophthalmologic examinations such as fluorescein fundus angiography and optical coherence tomography. Treatment methods for the disease include laser photocoagulation, photodynamic therapy (PDT), and intravitreal injection of anti-VEGF agents.

Such age-related macular degeneration has no initial subjective symptoms and is often mistaken for other causes. Thus, there are problems in that it is not only difficult to detect the disease in the early stage but also expensive equipment is required for its diagnosis. In addition, since the diagnostic methods described above are very inconvenient and dangerous to carry out, subjects are reluctant to undergo such methods. Therefore, there is a demand for development of a test method capable of diagnosing the likelihood of occurrence or presence of age-related macular degeneration in a simple and quick manner.

Meanwhile, clonal hematopoiesis (CH) is a condition defined as expansion of clone-derived hematopoietic stem cells (HSCs) carrying a somatic mutation in leukemia-related genes, which can be detected by next generation sequencing (NGS) (see Genovese G, Kahler AK, Handsaker RE, et al: Clonal hematopoiesis and blood-cancer risk inferred from blood DNA sequence. N Engl J Med 371:2477-87, 2014; Park SJ, Bejar R: Clonal hematopoiesis in cancer. Exp Hematol 83:105-112, 2020; Jaiswal S, Ebert BL: Clonal hematopoiesis in human aging and disease. Science 366, 2019). It has been reported that occurrence of CH is associated with aging and is significantly associated with development of cardiovascular diseases and hematological malignancies.

### Disclosure of Invention

### Technical Problem

An object of the present invention is to solve the problems of the prior art as described above.

Another object of the present invention is to provide a biomarker for predicting occurrence of, diagnosing, or treating age-related macular degeneration.

Yet another object of the present invention is to provide a composition, a kit, or a panel for predicting occurrence of, diagnosing, or treating age-related macular degeneration.

Still yet another object of the present invention is to provide a method for providing information for predicting occurrence of, diagnosing, or treating age-related macular degeneration.

### Solution to Problem

In order to achieve the above-described objects, the present inventors have studied to obtain a biomarker for early diagnosis of age-related macular degeneration. As a result, the present inventors have identified that presence of a clonal hematopoiesis (CH)-inducing gene mutation(s) is an important factor, thereby completing the present invention.

In an aspect of the present invention, there is provided a composition for providing information necessary for predicting occurrence of, diagnosing, or treating age-related macular degeneration, the composition comprising an agent(s) capable of detecting a clonal hematopoiesis-inducing mutation(s) using a biological sample isolated from a subject.

In another aspect of the present invention, there is provided a kit for providing information necessary for predicting occurrence of, diagnosing, or treating age-related macular degeneration, the kit comprising the composition.

In yet another aspect of the present invention, there is provided a genetic analysis panel for providing information necessary for predicting occurrence of, diagnosing, or treating age-related macular degeneration, the panel comprising the composition.

In still yet another aspect of the present invention, there is provided a method for providing information for predicting occurrence of, diagnosing, or treating age-related macular degeneration, the method comprising determining whether a clonal hematopoiesis-inducing mutation(s) exists in a subject through genetic analysis of a biological sample isolated from the subject.

### Advantageous Effects of Invention

The marker composition, the kit, the panel, and the method for providing information for predicting occurrence of, diagnosing, or treating age-related macular degeneration, according to the present invention, are novel tools capable of diagnosing, preventing, or treating age-related macular degeneration, which not only have excellent sensitivity but also allow for convenient analysis without using a biopsy, so that they can be particularly effectively used for early diagnosis, prevention, or treatment of age-related macular degeneration.

### Brief Description of Drawings

FIG. 1 illustrates, as a bar graph, detection frequencies of respective genes showing a somatic variant with a VAF of 1.5% or higher identified in Example 3.1.1.
FIG 2 illustrates, as a bar graph, detection frequencies of respective genes showing a somatic variant with a VAF of 2% or higher identified in Example 4.1.1.

### Best Mode for Carrying Out Invention

Hereinafter, the present invention will be described in detail.

In an aspect of the present invention, there is provided a composition for providing information necessary for predicting occurrence of, diagnosing, or treating age-related macular degeneration, the composition comprising an agent(s) capable of detecting a clonal hematopoiesis-inducing mutation(s) using a biological sample isolated from a subject.

As used herein, the term "clonal hematopoiesis" refers to a phenomenon in which, when hematopoietic stem cells have undergone a somatic mutation(s) to gain an opportunity for selective proliferation, mutated clones expand and take up a certain portion of white blood cells. Genes (with a clonal hematopoiesis-inducing mutation(s)), in which a somatic mutation(s) associated with clonal hematopoiesis occurs, include APC, ASXL1, ASXL2, ATM, BCL11B, BCOR, BCORL1, BIRC3, BRAF, BRCC3, CARD11, CASP8, CBL, CD58, CD79B, CNOT3, CREBBP, CUX1, DDX3X, DNMT3A, EP300, ETV6, EZH2, FAM46C, FBXW7, FLT3, FOXP1, GNAS, GNB1, GPS2, HIST1H1C, IDH2, IKZF1, IKZF2, JAK1, JAK2, JAK3, JARID2, KDM6A, KIT, KLHL6, KMT2D, KRAS, LUC7L2, MAP3K1, MPL, MYD88, NF1, NFE2L2, NOTCH1, NOTCH2, NRAS, PDS5B, PDSS2, PHF6, PHIP, PIK3CA, PIK3R1, PPM1D, PRDM1, PRPF40B, PTEN, PTPN11, RAD21, RIT1, RPS15, SETD2, SETDB1, SF1, SF3A1, SF3B1, SMC1A, SMC3, SRSF2, STAG1, STAG2, STAT3, SUZ12, TBL1XR1, TET1, TET2, TNFAIP3, TNFRSF14, TP53, U2AF1, VHL, WT1, ZRSR2, and CHEK2.

In an embodiment, the clonal hematopoiesis-inducing mutation(s) may be or comprise a mutation(s) in one or more genes selected from the group consisting of APC, ASXL1, ASXL2, BCOR, CD58, CHEK2, CUX1, DNMT3A, EP300, EZH2, GNB1, JAK1, JAK2, JARID2, KMT2D, NF1, NOTCH2, PPM1D, RIT1, SETD2, SF1, SF3B1, SRSF2, STAG1, STAT3, SUZ12, TBL1XR1, TET2, TNFAIP3, and U2AF1.

In another embodiment, the mutation(s) may be or comprise a mutation(s) in one or more genes selected from the group consisting of APC, ASXL1, ASXL2, CD58, CHEK2, CUX1, DNMT3A, EP300, EZH2, GNB1, JAK1, JAK2, JARID2, KMT2D, NF1, NOTCH2, PPM1D, RIT1, SETD2, SF1, SF3B1, SRSF2, STAT3, SUZ12, TBL1XR1, TET2, TNFAIP3, and U2AF1.

In yet another embodiment, the mutation(s) may be or comprise a mutation(s) in one or more genes selected from the group consisting of DNMT3A, TET2, ASXL1, APC, ASXL2, BCOR, CHEK2, CUX1, EP300, EZH2, GNB1, JAK1, JAK2, KMT2D, NF1, NOTCH2, RIT1, SETD2, SF3B1, SRSF2, STAG1, STAT3, SUZ12, and TNFAIP3.

In still yet another embodiment, the mutation(s) may be or comprise a mutation(s) in one or more genes selected from the group consisting of DNMT3A, TET2, ASXL1, SETD2, KMT2D, NF1, NOTCH2, SF3B1, ASXL2, CHEK2, CUX1, EZH2, GNB1, JAK1, JAK2, RIT1, SRSF2, SUZ12, APC, STAT3, and TNFAIP3.

In still yet another embodiment, the mutation(s) may be or comprise a mutation(s) in one or more genes selected from the group consisting of DNMT3A, TET2, and ASXL1.

In still yet another embodiment, the mutation(s) may be or comprise a mutation(s) in DNMT3A.

In still yet another embodiment, the mutation(s) may be or comprise a mutation(s) in TET2.

In still yet another embodiment, the mutation(s) may be or comprise a mutation(s) in DNMT3A and TET2, or DNMT3A, TET2, and ASXL1.

A disease type of age-related macular degeneration, for which the composition can provide information necessary for predicting occurrence of, diagnosing, or treating the disease, may include dry-type and/or wet-type. The dry-type age-related macular degeneration may develop into wet-type age-related macular degeneration as lesions such as drusen or retinal pigment epithelium atrophy further progress in the retina.

As used herein, the term "predicting occurrence" means selecting or identifying, among subjects who have not been diagnosed with age-related macular degeneration by clinical symptoms, a subject who has an increased tendency or risk of developing age-related macular degeneration or has such a tendency or risk at a relatively high level.

As used herein, the term "diagnosing" or "treating" means diagnosing or treating a disease or condition as used in its conventional sense in the art. As used herein, the term "diagnosing" is meant to include determining susceptibility of a subject, that is, a test subject, to age-related macular degeneration, determining whether a subject currently has an age-related macular degeneration disease or condition, or monitoring a subject's status following treatment in order to provide information on efficacy of the treatment on age-related macular degeneration. In the narrowest sense, it means identifying whether age-related macular degeneration has developed. In addition, it includes providing early diagnosis for prevention or treatment of age-related macular degeneration or providing information, such as genetic information, for early diagnosis of age-related macular degeneration.

As used herein, the term "subject" refers to a mammal, including a human, but is not limited thereto.

As used herein, the term "biological sample" refers to any biological specimen obtained from a subject and includes, but is not limited to, samples, such as blood, serum, plasma, lymph fluid, saliva, sputum, mucus, urine, or feces, isolated from a subject for whom identification needs to be made whether age-related macular degeneration has developed.

In an embodiment, the mutation(s) may lower or lack activity of the protein encoded by a listed gene as compared with its wild type. In addition, the mutation(s) may be a somatic mutation.

The mutation(s) may be in the form of a missense mutation, a frameshift mutation, a nonsense mutation or a splice mutation, insertion, deletion or substitution of nucleotide(s), combinations thereof, or the like.

As used herein, the term "missense mutation" refers to a genetic mutation in which a single base substitution occurs at a certain site on its DNA chain so that the genetic code of mRNA changes and designates a different amino acid from the original one, thereby affecting the resulting protein.

As used herein, the term "frameshift mutation" refers to a genetic mutation caused by insertion or deletion of the number of bases that is not divisible by three.

As used herein, the term "nonsense mutation" refers to a genetic mutation in which, due to a single base substitution, a codon encoding an original amino acid is changed to a stop codon that does not encode an amino acid so that protein synthesis stops at a site where the codon is located.

As used herein, the term "splice mutation" refers to a mutation caused by use of an alternative splicing site within a transcribed RNA molecule or between individually transcribed RNA molecules.

For example, the mutation(s) in DNMT3A gene may be, but is not limited to, one or more selected from the mutations listed in Table 1.

**[Table 1]**

| **Gene** | **Refseq** | **ExonIndex** | **Effect** | **AA** | **CDS** |
|---|---|---|---|---|---|
| DNMT3A | NM_022552.4 | 20 | NON_SYNONYMOUS_CODING | p.Phe794Leu | c.2382C>G |
| DNMT3A | NM_022552.4 | 16 | NON_SYNONYMOUS_CODING | p.Val636Met | c.1906G>.A |
| DNMT3A | NM_022552.4 | 19 | NON_SYNONYMOUS_CODING | p.Tyr735Cys | c.2204A>G |
| DNMT3A | NM_022552.4 | 14 | NON_SYNONYMOIJS_CODING | p.Cys540Tyr | c.1619G>A |
| DNMT3A | NM_022552.4 | 13 | NON_SYNONYMOUS_CODING | p.Cys494Ser | c.1480T>A |
| DNMT3A | NM_022552.4 | 17 | NON_SYNONYMOUS_CODING | p.Leu653Phe | c.1959G>C |
| DNMT3A | NM_022552.4 | 19 | NON_SYNONYMOUS_CODING | p.Pro743Leu | c.2228C>T |
| DNMT3A | NM_022552.4 | 17 | SPLICE_SITE_DONOR | - | - |
| DNMT3A | NM_022552.4 | 7 | STOP_GAINED | p.Gln248* | c.742C>T |
| DNMT3A | NM_022552.4 | 8 | FRAME_SHIFT | p.314Met_315Thrfs | c.944_945insTAGGTGGT |
| DNMT3A | NM_022552.4 | 15 | SPLICE_SITE_DONOR | | |
| DNMT3A | NM_022552.4 | 21 | STOP_GAINED | p.Glu817* | c.2449G>T |
| DNMT3A | NM_022552.4 | 22 | FRAME_SHIFT | p.840Lys_841GInfs | c.2522_2523insA |
| DNMT3A | NM_022552.4 | 10 | NON_SYNONYMOUS_CODING | p.Arg379His | c.1136G>A |
| DNMT3A | NM_022552.4 | 8 | NON_SYNONYMOUS_CODING | p_Arg326Leu | c.977G>T |
| DNMT3A | NM_022552.4 | 19 | NON_SYNONYMOUS_CODING | p.Pro743Arg | c.2228C>G |
| DNMT3A | NM_022552.4 | 19 | NON_SYNONYMOUS_CODING | p.Phe732Ile | c.2194T>A |
| DNMT3A | NM_022552.4 | 15 | STOP_GAINED | p.Tyr592* | c.1776C>G |
| DNMT3A | NM_022552.4 | 10 | FRAME_SHIFT | p.398Val_399Glufs | c.1195_1196delG |
| DNMT3A | NM_022552.4 | 20 | NON_SYNONYMOUS_CODING | p.Gly796Asp | c.2387G>A |
| DNMT3A | NM_022S52.4 | 8 | NON_SYNONYMOUS_CODING | p.Gly293Val | c.878G>T |
| DNMT3A | NM_022552.4 | 19 | NON_SYNONYMOUS_CODING | p.Arg749Cys | c.2245C>T |
| DNMT3A | NM_022552.4 | 23 | NON_SYNONYMOUS_CODING | p.Tyr908Asp | c.2722T>G |
| DNMT3A | NM_022552.4 | 15 | NON_SYNONYMOUS_CODING | p.Cys583Ser | c.1748G>C |
| DNMT3A | NM_022552.4 | 18 | NON_SYNONYMOUS_CODING | p.Ile705Val | c.2113A>G |
| DNMT3A | NM_022552.4 | 19 | NON_SYNONYMOUS_CODING | p.Glu774Lys | c.2329G>A |
| DN MT3A | NM_022552.4 | 19 | NON_SYNONYMOUS_CODING | p.Arg749Cys | c.2245C>T |
| DN MT3A | NM_0225524 | 13 | NON_SYNONYMOUS_CODING | p.Cys517Trp | c.1551C>G |
| DN MT3A | NM_022552.4 | 18 | FRAME_SHIFT | p.706Gly_707Serfs | c_2120 2121deIG |
| DN MT3A | NM_022552.4 | 23 | NON_SYNONYMOUS_CODING | p.Arg882Ser | c.2644C>A |
| DNMT3A | NM_022552.4 | 19 | STOP_GAINED | p.Ser770* | c.2309C>A |
| DNMT3A | NM_022552.4 | 23 | NON_SYNONYMOUS_CODING | p.Tyr908Asp | c.2722T>G |
| DNMT3A | NM_022552.4 | 7 | FRAME_SHIFT | p.232Pro_233Glyfs | c.699_700deIC |
| DN MT3A | NM_022552.4 | 23 | NON_SYNONYMOUS_CODING | p.Arg882His | c.2645G>A |
| DN MT3A | NM_022552.4 | 7 | SPLICE_SITE_DONOR | - | - |
| DN MT3A | NM_022552.4 | 8 | NON_SYNONYMOUS_CODING | p.Gly298Arg | c.892G>C |
| DN MT3A | NM_022552.4 | 20 | NON_SYNONYMOUS_CODING | p.Met801Thr | c.2402T>C |
| DN MT3A | NM_022552.4 | 22 | FRAME_SHIFT | p.854Lys_855Glufs | c.2564_2565delAA |
| DN MT3A | NM_022552.4 | 17 | STOP_GAINED | p.Ser663* | c.1988C>A |

The mutation(s) in TET2 gene may be, but is not limited to, one or more selected from the mutations listed in Table 2.

**[Table 2]**

| **Gene** | **Refseq** | **ExonIndex** | **Effect** | **AA** | **CDS** |
|---|---|---|---|---|---|
| TET2 | NM_0011272082 | 3 | FRAME_SHIFT | p.281Ser_282Glufs | c.846_347insT |
| TET2 | NM_001127208.2 | 11 | FRAME_SHIFT | p.1856Pro_1859Glyfs | c.5570_5573deICTGACAT |
| TET2 | NM_001127208.2 | 11 | NON_SYNONYMOUS_CODING | p.Arg1926Cys | c.5776C>T |
| TET2 | NM_0011272082 | 6 | STOP_GAINED | p.Arg1216* | c.3646C>T |
| TET2 | NM_001127208.2 | 7 | NON_SYNONYMOUS_CODING | p.Ala1283Pro | c.3847G>C |
| TFT2 | NM_001127208.2 | 11 | NON_SYNONYMOUS_CODING | p.Ile1873Thr | c.5618T>C |
| TET2 | NM_001127208.2 | 11 | FRAME_SHIFT | p.1560Ser_1561Alafs | c.4681.4682insC |
| TET2 | NM_001127208.2 | 11 | STOP_GAINED | p.Glu1826* | c.5476G>T |
| TET2 | NM_001127208.2 | 11 | NON_SYNONYMOUS_CODING | p.Leu1646Pro | c.4937T>C |
| TET2 | NM_001127208.2 | 11 | FRAME_SHIFT | p.1644Tyr_1645Leufs | c.4935_4936delT |
| TET2 | NM_001127208.2 | 4 | NON_SYNONYMOUS_CODING | p.Arg1161Gly | c.3481A>G |
| TET2 | NM_001127208.2 | 3 | FRAME_SHIFT | p.1068Thr_1069Thrfs | c.3206_3207delC |
| TET2 | NM_001127208.2 | 3 | STOP_GAINED | p.Glu186* | c.556G>T |
| TET2 | NM_001127208.2 | 3 | FRAME_SHIFT | p.346Gln_847Thrfs | c.2540.2541insA |
| TET2 | NM_001127208.2 | 3 | FRAME_SHIFT | p_1102Asn_1103Phefs | c.3309_3310deIT |
| TET2 | NM_001127208.2 | 6 | STOP_GAINED | p.Tyr1255* | c.3765C>G |
| TET2 | NM_0011272082 | 8 | NON_SYNONYMOUS_CODING | p.Tyr1345Cys | c.4034A>G |
| TET2 | NM_001127208.2 | 11 | FRAME_SHIFT | p.1543Pro_1544Glnfs | c.46300_4631insC |
| TET2 | NM_001127208.2 | 7 | NON_SYNONYMOUS_CODING | p.Phe1309Leu | c.3927T>G |
| TET2 | NM_001127208.2 | 3 | FRAME_SHIFT | p.131Asn_132Profs | c.396_397delT |
| TET2 | NM_001127208.2 | 10 | FRAME_SHIFT | p.1483_1484Serfs | c.4452_4453deIC |

The mutation(s) in ASXL1 gene may be, but is not limited to, one or more selected from the mutations listed in Table 3.

**[Table 3]**

| **Gene** | **Refseq** | **ExonIndex** | **Effect** | **AA** | **CDS** |
|---|---|---|---|---|---|
| ASXL1 | NM_015338.5 | 12 | FRAME_SHIFT | p.642Gly_643Glyfs | c.1927_1928insG |
| ASXL1 | NM_015338.5 | 12 | FRAME_SHIFT | p.816Leu_317Valfs | c.2451_2452deIA |
| ASXL1 | NM_015338.5 | 12 | FRAME_SHIFT | p.975Gln_976Glnfs | c.2927_2928insA |
| ASXL1 | NM_015338.5 | 12 | STOP_GAINED | p.Glu790* | c.2368G>T |
| ASXL1 | NM_015338.5 | 12 | FRAME_SHIFT | p.787Glu_788Cysfs | c.2363_2364delA |
| ASXL1 | NM_015338.5 | 12 | FRAME_SHIFT | p.642Gly_643Glyfs | c.1927_1928insG |
| ASXL1 | NM_015338.5 | 12 | FRAME_SHIFT | p.830Leu_831Aspfs | c.2492_2493insT |
| ASXL1 | NM_015338.5 | 12 | STOP_GAINED | p.Gln760* | c.2278C>T |
| ASXL1 | NM_015338.5 | 12 | STOP_GAINED | p.Gln882* | c.2644C>T |

The mutation(s) in APC, ASXL2, BCOR, CD58, CHEK2, CUX1, EP300, EZH2, GNB1, JAK1, JAK2, JARID2, KMT2D, NF1, NOTCH2, PPM1D, RIT1, SETD2, SF1, SF3B1, SRSF2, STAG1, STAT3, SUZ12, TBL1XR1, TNFAIP3, and U2AF1 genes may be, but is not limited to, the mutation(s) listed in Table 4.

**[Table 4]**

| **Gene** | **Refseq** | **ExonIndex** | **Effect** | **AA** | **CDS** |
|---|---|---|---|---|---|
| APC | NM_001127510.2 | 17 | NON_SYNONYMOUS_CODING | p.Ser2390Asn | c.7169G>A |
| APC | NM_001127510.2 | 13 | NON_SYNONYMOUS_CODING | p.Ala501Val | c.1502C>T |
| APC | NM_001127510.2 | 9 | NON_SYNONYMOUS_CODING | p.Arg259Gln | c.776G>A |
| ASXL2 | NM_018263.4 | 12 | STOP_GAINED | p.Cys1428* | c.4284C>A |
| BCOR | NM_001123385.1 | 4 | NON_SYNONYMOUS_CODING | p.Pro904Leu | c.2711C>T |
| CD58 | NM_001779.2 2 | | NON_SYNONYMOUS_CODING | p.Phe116Tyr | c.347T>A |
| CHEK2 | NM_0D10057:35.1 | 4 | NON_SYNONYMOUS_CODING | p.Arg188Trp | c.562C>T |
| CUX1 | NM_001202543.1 | 23 | NON_SYNONYMOUS_CODING | p.Leu1294Pro | c.3381T>C |
| EP300 | NM_001429.3 | 22 | NON_SYNONYMOUS_CODING | p.Arg1252Thr | c.3755G>C |
| EP300 | NM_001429.3 | 29 | NON_SYNONYMOUS_CODING | p.Ala1586Ser | c.4756G>T |
| EZH2 | NM_004456.4 | 16 | NON_SYNONYMOUS_CODING | p.Gly628Arg | c.1882G>C |
| EZH2 | NM_004456.4 | 6 | NON_SYNONYMOUS_CODING | p.Asp192Asn | c.574G>A |
| GNB1 | NM_001282539.1 | 4 | NON_SYNONYMOUS_CODING | p.Lys57Glu | c169A>G |
| JAK1 | NM_002227.2 | 24 | NON_SYNONYMOUS_CODING | p.Gly1097Ser | c.3289G>A |
| JAK2 | NM_004972.3 | 14 | NON_SYNONYMOUS_CODING | p.Val617Phe | c.1849G>T |
| JAK2 | NM_004972.3 | 25 | FRAME_SHIFT | p.1127Asp_1128Asnfs | c.3383_3384insA |
| JARID2 | NM_004973.3 | 18 | NON_SYNONYMOUS_CODING | p.Arg1221Pro | c.3662G>C |
| KMT2D | NM_003482.3 | 10 | NON_SYNONYMOUS_CODING | p.Ser504Phe | c.1511C>T |
| KMT2D | NM_003482.3 | 31 | NON_SYNONYMOUS_CODING | p.Asn2517Ser | c.7550A>G |
| NF1 | NM_001042492.2 | 16 | FRAME_SHIFT | p.577Leu_578Phefs | c.1733_1734deIT |
| NF1 | NM_001042492.2 | 17 | STOP_GAINED | p.Tyr628* | c.1884C>A |
| NOTCH2 | NM_024408.3 | 34 | NON_SYNONYMOUS_CODING | pArg2400Gln | c.7199G>A |
| NOTCH2 | NM_024408.3 | 4 | NON_SYNONYMOUS_CODING | p_Asn232Ser | c.695A>G |
| NOTCH2 | NM_024408.3 | 34 | NON_SYNONYMOUS_CODING | p.Arg2453Trp | c.7357C>T |
| NOTCH2 | NM_024408.3 | 30 | NON_SYNONYMOUS_CODING | p.His1793Gln | c.5379C>A |
| PPM1D | NM_003620.3 | 6 | STOP_GAINED | p.Arg581* | c.1741C>T |
| RIT1 | NM_001256821.1 | 5 | NON_SYNONYMOUS_CODING | p.Gly112Ala | c.335G>C |
| SETD2 | NM_014159.6 | 3 | NON_SYNONYMOUS_CODING | p.Cys805Tyr | c.2414G>A |
| SETD2 | NM_014159.6 3 | | NON_SYNONYMOUS_CODING | p.Pro215Leu | c.644C>T |
| SF1 | NM_001178030.1 | 10 | NON_SYNONYMOLlS_CODING | p.Pro525Ser | c.1573C>T |
| SF3B1 | NM_012433.2 | 1 | NON_SYNONYMOUS_CODING | p.Ala5Thr | c.13G>A |
| SRSF2 | NM_003016.4 | 1 | NON_SYNONYMOUS_CODING | p.Pro95Leu | c.284C>T |
| STAG 1 | NM_005862.2 | 25 | NON_SYNONYMOUS_CODING | p.Ile876Thr | c.2627T>C |
| STATS | NM_139276.2 | 20 | NON_SYNONYMOUS_CODING | p.Gly618Arg | c.1852G>C |
| SUZ12 | NM_015355.2 | 15 | NON_SYNONYMOUS_CODING | p.Phe603Cys | c.1808T>G |
| TBL1XR1 | NM_024665.4 | 6 | NON_SYNONYMOUS_CODING | p.Glu171Asp | c.513A>C |
| TNFAIP3 | NM_001270507.1 | 2 | FRAME_SHIFT | p.35Ile_37Hisfs | c.107_10.9delTCAT |
| TNFAIP3 | NM_001270507.1 | 8 | STOP_GAINED | p.Cys662* | c.1986C>A |
| U2AF1 | NM_001025203.1 | 8 | NON_SYNONYMOUS_CODING | p.Gly217Ser | c.649G>A |

For example, the mutation in exon 14 of JAK2 gene may be a missense mutation in which the base G at position 1849 is substituted with T in the nucleotide sequence represented by NM_004972.3.

In an embodiment, the agent(s) capable of detecting the mutation(s) may include, for example, agents capable of detecting the mutated gene, mRNA derived therefrom, or expression of the protein encoded by the mutated gene.

The agent(s) capable of detecting the mutated gene or expression of its mRNA may be, but is not limited to, a nucleotide sequence that binds complementarily to the gene or its mRNA, for example, a sense and antisense primer set, a probe, or antisense nucleic acid.

As used herein, the term "probe" refers to a substance capable of specifically binding to a target substance to be detected in a sample, in which presence of the target substance in the sample can be specifically identified through the binding. The probe may be prepared in the form of an oligonucleotide probe, a single-stranded DNA probe, a double-stranded DNA probe, an RNA probe, or the like. Probe selection and hybridization conditions may be modified based on those known in the art.

As used herein, the term "primer" refers to a nucleic acid sequence capable of forming a base pair with its complementary template and functioning as a starting point for copying the template strand. A sequence of the primer does not necessarily have to be exactly the same as a sequence of the template, and only needs to be sufficiently complementary to the template so that it can hybridize therewith. The primer enables initiation of DNA synthesis in the presence of reagents for polymerization and four different nucleoside triphosphates in an appropriate buffer solution and at an appropriate temperature. PCR conditions and lengths of sense and antisense primers may be modified based on those known in the art. For example, it is possible to design the primer using a commercially available program for primer design.

As used herein, the term "antisense nucleic acid" refers to a nucleic acid-based molecule that has a nucleotide sequence complementary to a targeted gene variant and is capable of forming a dimer therewith. The antisense nucleic acid may be complementary to the polynucleotide or a fragment thereof, or both of them. The antisense nucleic acid may have a length of 10 nts or longer, more specifically 10 to 200 nts, 10 to 150 nts, or 10 to 100 nts, and may be selected to have an appropriate length for increased detection specificity.

Using the primer, the probe, or the antisense nucleic acid, it is possible to amplify or identify presence of a nucleotide sequence having a specific allele at a mutation site.

As an example of the agent, the following probe sequence information may be listed.

**[Table 5]**

| **No** | **Chromosome** | **Start** | **Stop** | **Gene** | **RefMrnaID** | **Exon_index** | **Strand** | **TotalExons** | **GC Percent** | **Probe_Sequence** |
|---|---|---|---|---|---|---|---|---|---|---|
| 1 | chr2 | 25457208 | 25457328 | DNMT3A | NM_022552 | 23 | - | 23 | 59.17 | CCAGCACTCACCCTGCCCTCTCTGCCTTTTCTCCC CCAGGGTATTTGGTTTCCCAGTCCACTATACTGAC GTCTCCAACATGAGCCGCTTGGCGAGGCAGAGA CTGCTGGGCCGGTCATG |
| 2 | chr2 | 25457208 | 25457328 | DNMT3A | NM_022552 | 23 | - | 23 | 59.17 | CCAGCACTCACCCTGCCCTCTCTGCCTTTTCTCCC CCAGGGTATTTGGTTTCCCAGTCCACTATACTGAC GTCTCCAACATGAGCCGCTTGGCGAGGCAGAGA CTGCTGGGCCGGTCATG |
| 3 | chr2 | 25453574 | 25458694 | DNMT3A | NM_022552 | 22 | - | 23 | 43.33 | TTCAGCAAAGTGAGGACCATTACTACGAGGTCAA ACTCCATAAAGCAGGGCAAAGACCAGCATTTTCC TGTCTTCATGAATGAGAAAGAGGACATCTTATGGT GCACTGAAATGGAAAGG |
| 4 | chr2 | 25459119 | 25459899 | DNMT3A | NM_022552 | 21 | - | 23 | 54.17 | TTACAGTCTCTCTTCTGCCTCCTAGGCCGTTGGCA TCCACTGTGAATGATAAGCTGGAGCTGCAGGAGT GTCTGGAGCATGGCAGGATAGCCAAGGTCAGCTC CAGCGTCTAGAACCTCT |
| 5 | thr2 | 25461981 | 25462101 | DNMT3A | NM_022552 | 20 | - | 23 | 52.5 | CGTCTCCTGTTTTGTAGTCCAACCCTGTGATGATTG ATGCCAAAGAAGTGTCAGCTGCACACAGGGCCC GCTACTTCTGGGGTAACCTTCCCGGTATGAACAG GTTGGTGAAAGCTCCTG |
| 6 | chr2 | 25461981 | 25462101 | DNMT3A | NM_022552 | 20 | - | 23 | 52.5 | CGTCTCCTGTTTTGTAGTCCAACCCTGTGATGATTG ATGCCAAAGAAGTGTCAGCTGCACACAGGGCCC GCTACTTCTGGGGTAACCTTCCCGGTATGAACAG GTTGGTGAAAGCTCCTG |
| 7 | chr2 | 25463125 | 25463245 | DNMT3A | NM_022552 | 19 | - | 23 | 51.67 | CCCTTCTTCTGGCTCTTTGAGAATGTGGTGGCCAT GGGCGTTAGTGACAAGAGGGACATCTCGCGATTT CTCGAGGTATAGCCAGCAACCTTGGTTTGGCCAG CTCACTAATGGCTTCTA |
| 8 | chr2 | 25463245 | 25463365 | DNMT3A | NM_022552 | 19 | - | 23 | 60 | CTATGCAGACAGCCCCAGCTGATGGCTTTCTCTTC CGACCTCTCAGAGGGCACTGGCCGGCTCTTCTTT GAGTTCTACCGCCTCCTGCATGATGCGCGGCCCA AGGAGGGAGATGATCGC |
| 9 | chr2 | 25463245 | 25463365 | DNMT3A | NM_022552 | 19 | - | 23 | 60 | CTATGCAGACAGCCCCAGCTGATGGCTTTCTCTTC CGACCTCTCAGAGGGCACTGGCCGGCTCTTCTTT GAGTTCTACCGCCTCCTGCATGATGCGCGGCCCA AGGAGGGAGATGATCGC |
| 10 | chr2 | 25463493 | 25463613 | DNMT3A | NM_002552 | 18 | - | 23 | 58.33 | CTTTATCCTCCCAGATCCAGGAGTGGGGCCCATT CGATCTGGTGATTGGGGGCAGTCCCTGCAATGAC CTCTCCATCGTCAACCCTGCTCGCAAGGGCCTCT ACGGTAGGTACCATCCTG |
| 11 | chr2 | 25464333 | 25464503 | DNMT3A | NM_022552 | 17 | - | 23 | 64.17 | CACGGTGGGCATGGTGCGGCACCAGGGGAAGAT CATGTACGTCGGGGACGTCCGCAGCGTCACACA GAAGCATGTATGTCCATGCTGTGGGGCGCAGCCC GTCTTCCCCTCCCTGCACAC |
| 12 | chr2 | 25464503 | 25464623 | DNMT3A | NM_022552 | 17 | - | 23 | 60 | CCAGGGAGATGGCTCCAAGTAACGGTGCTGTCTG CTGGCTGGTGCAGGGCTCCTGGTGCTGAAGGACT TGGGCATTCAGGTGGACCGCTACATTGCCTCGGA GGTGTGTGAGGACTCCAT |
| 13 | chr2 | 25464503 | 25464&23 | DNMT3A | NM_022552 | 17 | - | 23 | 60 | CCAGGGAGATGGCTCCAAGTAACGGTGCTGTCTG CTGGCTGGTGCAGGGCTCCTGGTGCTGAAGGACT TGGGCATTCAGGTGGACCGCTACATTGCCTCGGA GGTGTGTGAGGACTCCAT |
| 14 | chr2 | 25466995 | 25467115 | DNMT3A | NM_022552 | 15 | - | 23 | 64.17 | GGCACAAGGGTACCTACGGGCTGCTGCGGCGGC GAGAGGACTGGCCCTCCCGGCTCCAGATGTTCTT CGCTAATAACCACGACCAGGAATTTGTGAGTGCT GGGCCTGGGGCGCGGTCTC |
| 15 | chr2 | 25469447 | 25 4 695 67 | DNMT3A | NM_0225 52 | 10 | - | 23 | 62.5 | GTGCAGAACAAGCCCATGATTGAATGGGCCCTGG GGGGCTTCCAGCCTTCTGGCCCTAAGGGCCTGGA GCCACCAGAAGGTAAATGAGGGCACCCAGCTTT CTGGGACCCCTGCCCGCCA |
| 16 | chr2 | 25470418 | 25470538 | DNMT3A | NM_22552 | 8 | - | 23 | 61.67 | TTGGTGGATGACGGGCCGGAGCCGAGCAGCTGA AGGCACCCGCTGGGTCATGTGGTTCGGAGACGGC AAATTCTCAGTGGTAAGTTGTGGGGTTTGGCAGTA GCCTGGGGTGGGGGAAGG |
| 17 | chr2 | 25470418 | 25470538 | DNMT3A | NM_02552 | 8 | - | 23 | 61.b7 | TTGGTGGATGACGGGCCGGAGCCGAGCAGCTGA AGGCACCCGCTGGGTCATGTGGTTCGGAGACGGC AAATTCTCAGTGGTAAGTTGTGGGGTTTGGCAGTA GCCTGGGGTGGGGGAAGG |
| 18 | chr2 | 25470538 | 25470658 | DNMT3A | NM_022552 | a | - | 23 | 62.5 | GTGACCACTGTGTAATGATTTCTGCTCCTTGGGGC TCCAGGACGGCCGGGGCTTTGGCATTGGGGAGCT GGTGTGGGGGAAACTGCGGGGCTTCTCCTGGTGG CCAGGCCGCATTGTGTC |
| 19 | chr20 | 31022338 | 31022458 | ASXL1 | MM_0153338 | 12 | + | 12 | 69.17 | GACTGGCGCCAGGACCCTCGCAGACATTAAAGC CCGTGCTCTGCAGGTCCGAGGGGCGAGAGGTCA CCACTGCCATAGAGAGGCGGCCACCACTGCCAT CGGAGGGGGGGGTGGCCCGGG |
| 20 | chr20 | 31022698 | 31022818 | ASXL1 | NM_015338 | 12 | + | 12 | 60.83 | AAGCTGCCTACTACAGAGGGCTACAGTTGGACTC ACAGATGGGCTAGGAGATGCCTCCCAACTCCCC GTTGCTCCCACTGGGGACCAGCCATGCCAGGCCT TGCCCCTACTGTCCTCCCA |
| 21 | chr20 | 31022818 | 31022938 | ASXL1 | NM_015338 | 12 | + | 12 | 51.67 | AACCTCAGTAGCTGAGAGATTAGTGGAGCAGCCT CAGTTGCATCCGGATGTTAGAACTGAATGTGAGTC TGGCACCACTTCCTGGGAAAGTGATGATGAGGAG CAAGGACCCACCGTTCC |
| 22 | chr20 | 3102 3058 | 31023178 | ASXL1 | NM_015338 | 12 | - | 12 | 45.83 | ACCTGAATCCTCACCGACTGATTGCCTGCAGAAC AGAGCATTTGATGACGAATTAGGGCTTGGTGGCTC ATGCCCTCCTATGAGGGAAAGTGATACTAGACAA GAAAACTTGAAAACCAA |
| 23 | chr4 | 1.06E+08 | 1.06E+08 | TET2 | M_00112720 | 3 | + | 11 | 40.83 | TGTGAGTCCTGACTTTACACAAGAAAGTAGAGGG TATTCCAAGTGTTTGCAAAATGGAGGAATAAAAC GCACAGTTAGTGAACCTTCTCTCTCTGGGCTCCTT CAGATCAAGAAATTGAA |
| 24 | chr4 | 1.06E+08 | 1.06E+08 | TET2 | M_00112720 | 3 | - | 11 | 41.67 | ACAAGACCAAAAGGCTAATGGAGAAAGACGTAA CTTCGGGGTAAGCCAAGAAAGAAATCCAGGTGA AAGCAGTCAACCAAATGTCTCCGATTTGAGTGAT AAGAAAGAATCTGTGAGTTC |
| 25 | chr4 | 1.06E-08 | 1.06E+08 | TET2 | M_0112720 | 3 | + | 11 | 49.17 | GATCAATTCCGCACAGACCTCTAACTCTGAGCTG CCTCCAAAGCCAGCTGCAGTGGTGAGTGAGGCCT GTGATGCTGATGATGCTGATAATGCCAGTAAACTA GCTGCAATGCTAAATAC |
| 26 | chr4 | 1.06E+08 | 1.06E+08 | TET2 | M_00112720 12720 | 3 | + | 11 | 34.17 | TTGTTCAAACAATACACACCTAGTTTCAGAGAATA AAGAACAGACTACACATCCTGAACTTTTTGCAGG AAACAAGACCCAAAACTTGCATCACATGCAATAT TTTCCAAATAATGTGAT |
| 27 | chr4 | 1.06E+06 | 1.06E+08 | TET2 | M_00112720 | 3 | + | 11 | 39.17 | ACTTGATAGCCACACCCCAGCTTTAGAGCAGCAA ACAACTTCTTCAGAAAAGACACCAACCAAAAGA ACAGCTGCTTCTGTTCTCAATAATTTTATAGAGTCA CCTTCCAAATTACTAGA |
| 28 | chr4 | 1.06E+08 | 1.06E.+08 | TET2 | M_00112720 | 4 | + | 11 | 40 | AGGAGCAGGTCCTAATGTGGCAGCTATTAGAGAA ATCATGGAAGAAAGGTAATTAACGCAAAGGCAC AGGGCAGATTAACGTTTATCCTTTTGTATATGTCAG AATTTTTCCAGCCTTCA |
| 29 | chr4 | 1.06E+08 | 1.06E+08 | TET2 | M_00112720 | 6 | + | 11 | 55.83 | ATGGTGATCCACGCAGGTGGTTCGCAGAAGCAGC AGTGAAGAGAAGCTACTGTGTTTGGTGCGGGAGC GAGCTGGCCACACCTGTGAGGCTGCAGTGATTGT GATTCTCATCCTGGTGTG |
| 30 | chr4 | 1.06E+08 | 1.06E-08 | TET2 | M_00112720 | 6 | + | 11 | 55 | GGAAGGAATCCCGCTGTCTCTGGCTGACAAACTC TACTCGGAGCTTACCGAGACGCTGAGGAAATACG GCACGCTCACCAATCGCCGGTGTGCCTTGAATGA AGAGTAAGTGAAGCCCAG |
| 31 | chr4 | 1.06E+08 | 1.06E+08 | TET2 | IM_00112720 | 6 | - | 11 | 55 | GGAAGGAATCCCGCTGTCTCTGGCTGACAAACTC TACTCGGAGCTTACCGAGACGCTGAGGAAATACG GCACGCTCACCAATCGCCGGTGTGCCTTGAATGA AGAGTAAGTGAAGCCCAG |
| 32 | chr4 | 1.06E-08 | 1.06E+08 | TET2 | IM_00112720 | S | - | 11 | 34.17 | ATTCACTTTATACAGGAAGAGAAACTGGAGTCTC ATTTGCAAAACCTGTCCACTCTTATGGCACCAAC ATATAAGAAACTTGCACCTGATGCATATAATAATC AGGTAAGTTTAAATAAT |
| 33 | chr4 | 1.06E+08 | 1.06E+08 | TET2 | M_00112720 | 10 | + | 11 | 47.5 | CTCAGGAGGAGAAAAAACGGAGTGGTGCCATTC AGGTACTGAGTTCTTTTCGGCGAAAAGTCAGGATG TTAGCAGAGCCAGTCAAGACTTGCCGACAAAGG AAACTAGAAGCCAAGAAAG |
| 34 | chr4 | 1.06E+08 | 1.06E+08 | TET2 | IM_00112720 | 11 | + | 11 | 50.83 | GAGACCCCAGCAGCAGCAGCCACATCACCCTCA GACAGAGTCTGTCAACTCTTATTCTGCTTCTGGAT CCACCAATCCATACATGAGACGGCCCAATCCAG TTAGTCCTTATCCAAACTC |
| 35 | chr4 | 1.06E+08 | 1.06E-08 | TET2 | IM_00112720 | 11 | + | 11 | 51.67 | TCTGGATCCTGACATTGGGGGAGTGGCCGTGGCT CCAACTCATGGGTCAATTCTCATTGAGTGTGCAAA GCGTGAGCTGCATGCCACAACCCCTTTAAAGAAT CCCAATAGGAATCACCC |
| 36 | chr4 | 1.06E+08 | 1.06E+08 | TET2 | IM.00112720 | 11 | + | 11 | 49.17 | CCCCACCAGGATCTCCCTCGTCTTTTACCAGCAT AAGAGCATGAATGAGCCAAAACATGGCTTGGCTC TTTGGGAAGCCAAAATGGCTGAAAAAGCCCGTGA GAAAGAGGAAGAGTGTGA |
| 37 | chr1 | 65301073 | 65301193 | JAK1 | NM_002227 | 24 | - | 25 | 46.67 | ATAGTTGTTCCTGAAAATGATAGGCCCAACCCAT GGCCAGATGACAGTCACAAGACTTGTGAATACGT TAAAAGAAGGAAAACGCCTGCCGTGCCCACCTA ACTGTCCAGATGAGGTATC |
| 33 | chr9 | 507 3 63 1 | 507 3 301 | JAK2 | NM_G04972 | 14 | + | 25 | 35 | TTTTTTTTTTCCTTAGTCTTTCTTTGAAGCAGCAAGT ATGATGAGCAAGCTTTCTCACAAGCATTTGGTTTT AAATTATGGAGTATGTGTCTGTGGAGACGAGAGTA AGTAAAACTACAG |
| 39 | chr2 | 1.98E+08 | 1.98E+08 | SF381 | NM_012433 | 23 | - | 25 | 38.33 | TGACTTATAATGTAACAGCTTGTTGACCCATTTGTT TTTTTCAGCCCTCATGATGTATTGGCTACACTTCTG AACAACCTCAAAGTTCAAGAAAGGCAGAACAGA GTTTGTACCACTGTA |
| 40 | chr2 | 1.98E+08 | 1.98E+08 | SF381 | NM_012433 | 1 | - | 25 | 47.5 | GCCAGTTCCGTCTGTGTGTTCGAGTGGACAAAATG GCGAA GATCGCCAAGACTCACGAAGGTAAGCGG TCTTTCCCTGCTTACGTGTTTTCTTCGTTGCTAGCCT AATAAAAGCCTTTTTT |
| | | | | | | | | | | |
| 41 | chr5 | 1.12E+08 | 1.12E+08 | APC | NM_00112751 | 17 | + | 17 | 40.83 | ACATCTCCAGGTAGACAGATGAGCCAACAGAAC CTTACCAAACAAACAGGTTTATCCAAGAATGCCA GTAGTATTCCAAGAAGTGAGTCTGCCTCCAAAGG ACTAAATCAGATGAATAAT |
| 42 | chr2 | 25964844 | 25964964 | ASXL2 | NM_18263 | 12 | - | 12 | 52.5 | CGCTTTCTGCCATGATGATTGCATCGGCCCCTCCA AACTGTGCGTCTCCTGCCTTGTCGTTCGGTAATGA GACTAGAAAGAGATACACTGTAAAGGAGGGGGA AGGGAAGGGTTGACCAG |
| 33 | chr11 | 1.08E+08 | 1.08E+08 | ATM | NM_000051 | 45 | + | 63 | 43.33 | CACACTTAGCAGGTTGCAGGCCATTGGAGAGCTG GAAAGCATTGGGGAGCTTTTCTCAAGGTATGTAAT TCGTATGACTTTGTTATCCTAAAGTGCAGCTTTTCT GTTACCAATAGTGAC |
| 44 | chr22 | 29121173 | 29121293 | CHEK2 | IM_00125738 | 3 | - | 16 | 33.33 | CCACTGCTGAAAAGAACAGATAAATACCGAACA TACAGCAAGAAACACTTTCGGATTTTCAGGGTAG GTAATGAATACCCATGTATCTAGGAGAGCTGGTA ATTTGGTCATTGTTTTTAG |
| 45 | chr7 | 1.02E+08 | 1.02E+08 | CUX1 | IM_00120254 | 23 | + | 24 | 60.83 | AAACCATCGAAGACCTCGCCACCCAGCTCAACC TGAAAACCAGCACCGTCATCAACTGGTTCCACAA CTACAGGTACGACGGCTGGCTCACAGGGAGCGC CGGTCGGCCCAGGGGAAGGG |
| 46 | chr22 | 41560035 | 41560155 | EP300 | NM_001429 | 22 | + | 31 | 45.83 | GAATTGGCTCTGCTCTTCCAGGTTTGTTGAATGTAC AGAGTGCGGAAGAAAGATGCATCAGATCTGTGTC CTTCACCATGAGATCATCTGGCCTGCTGGGTAAGT CTTAACGTTGTTACT |
| 47 | chr7 | 1.49E+08 | 1.49E+08 | EZH2 | IM_00120324 | 16 | - | 20 | 43.33 | TTTATTCTCTAGCATCTATTGCTGGCACCATCTGAC GTGGCAGGCTGGGGGATTTTTATCAAAGATCCTGT GCAGAAAAATGAATTCATCTCAGAATACTGTGGA GAGGTAAGGCACTGA |
| 43 | chr1 | 1747187 | 1747307 | GNB1 | NM_002074 | 5 | - | 12 | 57.5 | TTCAAGATCACAAACAACATCGACCCAGTGGGA AGAATCCAAATGCGCACGAGGAGGACACTGCGG GGGCACCTGGCCAAGATCTACGCCATGCACTGG GGCACAGACTCCAGGTAGGCG |
| 49 | chr12 | 49433932 | 49434052 | KMT2D | NM_003482 | 31 | - | 54 | 63.33 | GGGCCAGCAGGTGAGCTCCATGCCAAGGTCCCA AGTGGGCAGCCCCCCAATTTTGTCCGGTCCCCTG GGACGGGTGCATTTGTGGGCACCCCCTCTCCCAT GCGTTTCACTTTCCCTCAG |
| 50 | chr12 | 49444397 | 49445017 | KMT2D | NM_003482 | 10 | - | 54 | 63.33 | CCTGTGCCTGAGGAGCCATGCTTGTCCCCCCAAC CTGAGGAATCACACCTGTCCCCCCAGTCTGAGGA GCCATGCCTGTCCCCCCGGCCTGAGGAATCGCAT CTGTCCCCTGAGCTTGAG |
| 51 | chr12 | 25398268 | 25398388 | KRAS | NM_0333360 | 2 | - | 6 | 35 | ATTAACCTTATGTGTGACATGTTCTAATATAGTCAC ATTTTCATTATTTTTATTATAAGGCCTGCTGAAAAT GACTGAATATAAACTTGTGGTAGTTGGAGCTGGTG GCGTAGGCAAGAG |
| 52 | chr17 | 29550403 | 29550523 | NF1 | IM_00104249 | 16 | + | 58 | 32.5 | TTATATCTGCATTAGGTTATTGATGATGCTAGTAAC AATGAACTTTATGTTACTGCAGCTCACAAATGCTT TTTTACATCTGCAAGAAATTAACTAGTCATCAAAT GCTTAGTAGCACAG |
| 53 | chr17 | 29552070 | 29552190 | NFt | IM_00104249 | 17 | + | 58 | 36.67 | TGTCAGTGCTTCAGTAAAGCTTATTTATTTATTTTTT TCTAGCAGGCAGATAGAAGTTCCTGTCACTTTCTC CTTTTTTACGGGGTAGGATGTGATATTCCTTCTAGT GGAAATACCAGT |
| 54 | hr1 | 1.2E+03 | 1.2E+08 | NOTCH2 | NM_024408 | 34 | - | 34 | 55.83 | TTCTCCCAGCCTATCATCCTTTCCCAGCCTCTGTG GGCAAGTACCCCACACCCCCTTCACAGCACAGT TATGCTTCCTCAAATGCTGCTGAGCGAACACCCA GTCACAGTGGTCACCTCC |
| 55 | chr1 | 1.2E+08 | 1.2E+08 | NOTCH2 | NM_024408 | 30 | - | 34 | 50.83 | GCCAGATTGATAGGGAGCATTGTTTTCACCTTTCA GGCTGAAGATGAGGCCTTACTCTCAGAAGAAGAT GACCCCATTGATCGACGGCCATGGACACAGCAG CACCTTGAAGCTGCAGAC |
| 56 | chr17 | 58740705 | 58740825 | PPM1D | NM_003620 | δ | + | 6 | 47.5 | ATTAGAAGAGTCCAATTCTGGCCCCCTGATGAAG AAGCATAGACGAAATGGCTTAAGTCGAAGTAGTG GTGCTCAGCCTGCAAGTCTCCCCACAACCTCACA GCGAAAGAACTCTGTTAA |
| 57 | chr17 | 58740705 | 58740825 | PPM1D | NM_003620 | 6 | + | 6 | 47.5 | ATTAGAAGAGTCCAATTCTGGCCCCCTGATGAAG AAGCATAGACGAAATGGCTTAAGTCGAAGTAGTG GTGCTCAGCCTGCAAGTCTCCCCACAACCTCACA GCGAAAGAACTCTGTTAA |
| 58 | chr12 | 50037867 | 50037987 | PRPF40B | IM_00103169 | 26 | + | 26 | 59.17 | CCTTTGCTCCAACAGACAGGCTGGGACACGTCAG AAAGTGAGCTGAGTGAGGGTGAGCTGGAGAGGC GGCGGCGGACACTCCTACAGCAGCTGGATGATC ACCAGTGACCCAATGAGCTG |
| 59 | chr12 | 50037867 | 50037987 | PRPF40B | NM_175736 | 26 | - | 26 | 59.17 | CCTTTGCTCCAACAGACAGGCTGGGACACGTCAG AAAGTGAGCTGAGTGAGGGTGAGCTGGAGAGGC GGCGGCGGACACTCCTACAGCAGCTGGATGATC ACCAGTGACCCAATGAGCTG |
| 60 | chr1 | 1.56E+08 | 1.56E+08 | RIT1 | NM_00125682 | 5 | - | 6 | 45.83 | ATAATGACCCTTGTTTCCCTCTAGGCAGAGTTTAC AGCCATGCGGGACCAGTATATGAGGGCAGGAGA AGGGTTTATCATCTGTTACTCTATCACGGATCGTC GAAGTTTCCATGAAGTT |
| 61 | chr3 | 47125660 | 47125780 | SETD2 | NM_014159 | 12 | - | 21 | 50 | TAAGACTGCTGTCCCTCCGTTGAGTGAAGGAGAT GGGTATTCTAGTGAGAATACATCGCGTGCTCATAC ACCACTCAACACACCTGATCCTTCCACCAAGCTG AGCACAGAAGCTGACAC |
| 62 | chr3 | 47163642 | 47163762 | SETD2 | NM_014159 | 3 | - | 21 | 34.17 | CAAAGATTCAGACATATACTGTACTTTGAACGATA GCAACCCTTCTTTGTGTAACTCTGAAGCTGAAAAT ATTGAGCCTTCAGTTATGAAGATTTCTTCAAATAG CTTTATGAATGTGCA |
| 63 | chr17 | 74732947 | 74733067 | SRSF2 | NR_036608 | 1 | - | 4 | 69.17 | TCGTTCGCTTTCACGACAAGCGCGACGCTGAGGA CGCTATGGATGCCATGGACGGGGCCGTGCTGGAC GGCCGCGAGCTGCGGGTGCAAATGGCGCGCTAC GGCCGCCCCCCGGACTCAC |
| 64 | chr3 | 1.36E+08 | 1.36E+08 | STAG1 | NM_0058.62 | 25 | - | 34 | 35 | CAGCAAACTTATCATTTATGACATTGTTGACATGC ATGCAGCTGCAGACATCTTCAAACACTACATGAA GGTATAGTTAAATATTCTTATTTTTCTCCTTCCTCTA ACTGGCAGAGAAAT |
| 65 | chr17 | 30323796 | 30323916 | SUZ12 | NM_01 5355 | 15 | + | 16 | 34.17 | TTTTTCTTTCTTTTTCCCAGCAAATTGAAGAGTTTTC TGATGTTAATGAAGGAGAGAAAGAAGTGATGAAA CTCTGGAATCTCCATGTCATGAAGCATGGGTAGG GTATTTCTAAATTAA |

This illustratively presents the probe sequence information for the chromosomal sequences, in which a somatic variant has been detected among the entire sequence of NGS panel, for the 24 genes of DNMT3A, TET2, ASXL1, APC, ASXL2, BCOR, CHEK2, CUX1, EP300, EZH2, GNB1, JAK1, JAK2, KMT2D, NF1, NOTCH2, RIT1, SETD2, SF3B1, SRSF2, STAG1, STAT3, SUZ12, and TNFAIP3, and the agent(s) for detecting a mutation(s) is not limited thereto. The probe sequences are represented by SEQ ID NOs: 1 to 65.

In addition, the agent capable of detecting a protein may be, but is not limited to, a monoclonal antibody, a polyclonal antibody, a chimeric antibody, a fragment (scFv) of each of these antibodies, or an aptamer, which is capable of specifically binding to the protein.

In another aspect of the present invention, there is provided a kit for providing information necessary for predicting occurrence of, diagnosing, or treating age-related macular degeneration, the kit comprising the composition.

Specifically, the kits may consist of one or more different component compositions, solutions, or devices suitable for assay methods. For example, the kit may be a reverse transcription polymerase chain reaction (RT-PCR) kit, a DNA chip kit, an enzyme-linked immunosorbent assay (ELISA) kit, a protein chip kit, or a rapid kit.

In yet another aspect of the present invention, there is provided a genetic analysis panel for providing information necessary for predicting occurrence of, diagnosing, or treating age-related macular degeneration, the panel comprising the composition.

The genetic analysis panel is a genetic variation test method, in which mutations for a plurality of target genes are contained in one panel. The genetic analysis panel may be based on NGS.

In still yet another aspect of the present invention, there is provided a method for providing information for predicting occurrence of, diagnosing, or treating age-related macular degeneration, the method comprising determining whether a clonal hematopoiesis-inducing mutation(s) exists in a subject through genetic analysis of a biological sample isolated from the subject.

For description of the "clonal hematopoiesis-inducing mutation," "subject," "biological sample," "age-related macular degeneration," "predicting the occurrence," "diagnosing," and "treating", reference is made to the foregoing.

Various statistical processing methods may be used to provide information necessary for predicting occurrence of, diagnosing, or treating age-related macular degeneration according to the present invention. As the statistical processing method, for example, a logistic regression analysis method may be used. In addition, it is possible to determine a level of confidence for significant difference between a test group and a control group through statistical processing in order to diagnose age-related macular degeneration. Data used for statistical processing are values obtained by performing duplicate, triplicate, or multiple analyses for each marker. This statistical analysis method is very useful for making a clinically significant determination through statistical processing of clinical and genetic data as well as biomarkers.

In an embodiment, in a case where a mutation(s) is identified in one or more genes selected from the group of genes in a subject, it is possible to determine that the subject has high incidence of age-related macular degeneration. Specifically, the method may further comprise determining that age-related macular degeneration is highly likely to occur in a case where mutation(s) exists in the one or more genes.

In addition, the method may further comprise determining that in a case where mutation(s) exists in the gene, it is necessary to perform a treatment in the subject so that the mutation(s) is suppressed or function of the gene, in which the mutation(s) exists, is restored or supplemented, in order to decrease risk of occurrence or progression of age-related macular degeneration. For example, through the above-described method, it is possible to provide information related to companion diagnostics of whether it is necessary to administer a specific therapeutic agent for age-related macular degeneration.

As used herein, the term "companion diagnostics" refers to one of the diagnostic tests to identify possibility of applying a specific therapeutic drug to a specific patient, and this means identifying or monitoring a subject to be treated for age-related macular degeneration through an agent(s) capable of detecting whether a clonal hematopoiesis-inducing mutation(s) exists or an experiment performed therewith.

The genetic analysis may be performed using next generation sequencing (NGS). For example, using the next generation sequencing, it is possible to analyze information generated through processing of data from whole genome sequencing, whole exome sequencing, RNA sequencing, or the like.

In still yet another aspect of the present invention, there is provided a method for screening a prophylactic or therapeutic substance for age-related macular degeneration, comprising testing a candidate substance for preventing or treating age-related macular degeneration in a cell line or animal model having the clonal hematopoiesis-inducing mutation(s) as described above.

Hereinafter, the present invention will be described in detail by way of examples. However, the following examples are only for illustrating the present invention, and the present invention is not limited to the following examples.

### Example 1. Patient group and sample collection

Blood samples were obtained from 197 patients aged 50 years or older and having age-related macular degeneration at the Ophthalmology Department of Seoul National University Hospital and 3278 normal controls aged 50 years or older at the Seoul National University Hospital Healthcare System Gangnam Center. This study was conducted with the approval of the Institutional Review Board (IRB) of Seoul National University Hospital (IRB No: 2001-151-1097).

### Example 2. Detection of somatic variant through next generation sequencing

In order to detect a somatic mutation in immune cells contained in the obtained blood samples, genomic DNA was extracted and subjected to next generation sequencing. To measure and detect a mutation that has proliferated in some of the immune cells, it is necessary to be able to detect a mutated nucleotide sequence that is not predetermined because the mutation may occur at various locations in dozens of genes. In addition, it is necessary to be able to reliably detect a minute mutation that is present at 1 to 2%. To this end, next generation sequencing (NGS) technology is the most optimal platform. A process for detecting a mutation in the immune cells is as follows.

### 1. Extraction of peripheral blood DNA

① 2 to 3 ml of peripheral blood is collected from a subject to be tested
② leukocyte fraction is separated by centrifugation
(3) DNA is extracted from the leukocyte fraction

### 2. Conversion of peripheral blood DNA into NGS library to allow for interpretation on NGS instrument

① Peripheral blood DNA is cut into appropriate lengths (150 to 300 base pairs) using ultrasound or restriction enzyme
② Adapter nucleotide sequence suitable for NGS instrument is attached to the cut DNA

### 3. Selection of target DNA using gene panel

① Hybridization with 89 gene probes is performed
② Only the hybridized DNA is selected and extracted

### 4. NGS sequencing and nucleotide sequence analysis

① The selected NGS library (targeted library) is input into NGS instrument
② Targeted amount of data is produced: Mean depth of coverage of 800x or higher
③ Mutated nucleotide sequence is identified as compared with a human reference genome (hg19)
④ Function of the identified nucleotide sequence is predicted and mutation database matching is performed
⑤ The results are analyzed

Here, in order to reliably detect a minute mutation at about 1 to 2%, it is necessary that NGS sequencing data has mean depth of coverage of 800x or higher, and noise is suppressed to a minimum.

In the present example, a method was applied in which a region to be sequenced is captured in a way of hybridization enrichment; and a sequencing library was prepared in a paired-end method (forward/reverse reads applied, read length = 150 base pairs) and produced using an Illumina sequencer (Nova-Seq). The NGS data includes all exons of the target genes as sequencing data, and the constructed NGS target panel (which performs sequencing only for specific genome sequences) included a total of 89 genes as follows:
APC, ASXL1, ASXL2, ATM, BCL11B, BCOR, BCORL1, BIRC3, BRAF, BRCC3, CARD11, CASP8, CBL, CD58, CD79B, CNOT3, CREBBP, CUX1, DDX3X, DNMT3A, EP300, ETV6, EZH2, FAM46C, FBXW7, FLT3, FOXP1, GNAS, GNB1, GPS2, HIST1H1C, IDH2, IKZF1, IKZF2, JAK1, JAK2, JAK3, JARID2, KDM6A, KIT, KLHL6, KMT2D, KRAS, LUC7L2, MAP3K1, MPL, MYD88, NF1, NFE2L2, NOTCH1, NOTCH2, NRAS, PDS5B, PDSS2, PHF6, PHIP, PIK3CA, PIK3R1, PPM1D, PRDM1, PRPF40B, PTEN, PTPN11, RAD21, RIT1, RPS15, SETD2, SETDB1, SF1, SF3A1, SF3B1, SMC1A, SMC3, SRSF2, STAG1, STAG2, STAT3, SUZ12, TBL1XR1, TET1, TET2, TNFAIP3, TNFRSF14, TP53, U2AF1, VHL, WT1, ZRSR2, and CHEK2.

NGS data was produced such that mean depth of coverage (DOC) was >= 800x. This was done by applying NGS data quality criteria which ensure that a minimum limit of detection (LOD) of a somatic variant is detected with variant allele frequency (VAF; also known as variant allele fraction) >= 1.5%. Detection of SNV, insertion, or deletion which constitutes the somatic variant was performed through a software for detection and analysis (in-house software) that had been implemented directly by the applicant and verified in several studies, and the following criteria were applied for determination of a valid somatic variant.
a) Sequence variant with a value of 1.5% <= VAF <= 30%
b) Presence of 5' and 3' reads, each of which is 5 or higher and provides evidence of sequence variant
c) Somatic variant, whose effect results in frameshift, stop codon gain, splice donor/acceptor, or amino acid change and which is detected once or higher for blood cancer or 10 times or higher for solid cancer in the oncogenomic database, is classified as potential driver (PD) and the other somatic variants are classified as non-PD.
d) Occurrence frequency of 0.2% or lower in all of 1000Genome project, ESP6500, and gnomAd
e) Only sequence variant is adopted which has an occurrence frequency of 2% or lower for non-PD or has confidence (99.9%) outside a false-positive VAF range even for PD, based on the in-house sequence detection frequency database

The sequence variants satisfying all of the above conditions were selected as valid somatic variants and applied to Example 3.

### Example 3. Data analysis I

### Example 3.1. Identification of genes showing prevalence

### Example 3.1.1. Prevalence analysis in patients having age-related macular degeneration

For the 197 samples from the patient group having age-related macular degeneration and the 3278 samples from the normal control group, prevalence of 89 genes was checked, and difference in the prevalence was analyzed by chi-square test. Here, a subject was defined as positive in a case where a genetic variation with variant allele fraction (VAF) of 1.5% or higher was detected in any one of the target genes. The analysis results are shown in Table 6.

**[Table 6]**

| Patient group having AMD, prevalence with VAF of 1.5 to 30% | | | |
|---|---|---|---|
| Ages | Number of samples | Positive (n) | Prevalence (%) |
| 50 | 15 | 3 | 20.0 |
| 60 | 69 | 21 | 30.4 |
| 70 | 92 | 35 | 38.0 |
| 80 | 21 | 12 | 57.1 |
| Total | 197 | 71 | 36.0 |

| Normal control group, prevalence with VAF of 1.5 to 30% | | | |
|---|---|---|---|
| Ages | Number of samples | Positive (n) | Prevalence (%) |
| 50 | 2006 | 340 | 16.9 |
| 60 | 1003 | 260 | 25.9 |
| 70 | 257 | 93 | 36.2 |
| 80 | 12 | 3 | 25.0 |
| Total | 3278 | 696 | 21.2 |

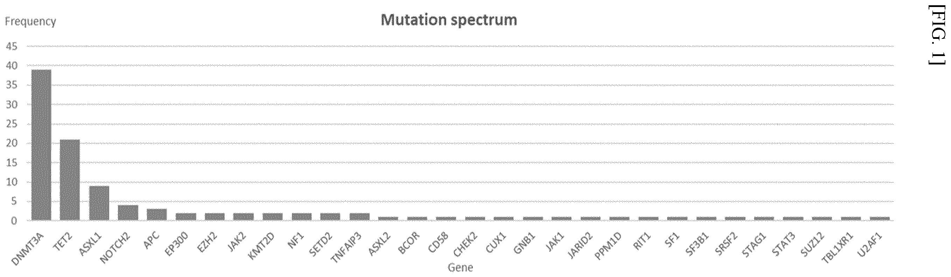

Referring to Table 6, it was identified that the prevalence for the group of 89 genes was significantly higher in the patient group having age-related macular degeneration than in the normal control group (36.0% vs 21.2%, p-value < 0.001). Among the group of 89 genes, genes showing a somatic variant with a VAF of 1.5% or higher were identified. As a result, a total of 30 genes were selected as follows: APC, ASXL1, ASXL2, BCOR, CD58, CHEK2, CUX1, DNMT3A, EP300, EZH2, GNB1, JAK1, JAK2, JARID2, KMT2D, NF1, NOTCH2, PPM1D, RIT1, SETD2, SF1, SF3B1, SRSF2, STAG1, STAT3, SUZ12, TBL1XR1, TET2, TNFAIP3, and U2AF1. Detection frequency of each gene is illustrated in FIG. 1. From the results, it can be seen that risk of age-related macular degeneration is significantly high in a case where a somatic variant exists in any one or more of the above genes.

### Example 3.1.2. Prevalence analysis in patients having wet-type age-related macular degeneration

The same analysis was performed on 153 samples of wet-type age-related macular degeneration among the 197 samples from the entire patient group having age-related macular degeneration which were analyzed in Example 3.1.1., and the analysis results are shown in Table 7.

**[Table 7]**

| Patient group having AMD, prevalence with VAF of 1.5 to 30% | | | |
|---|---|---|---|
| Ages | Number of samples | Positive (n) | Prevalence (%) |
| 50 | 6 | 2 | 33.3 |
| 60 | 60 | 21 | 27.2 |
| 70 | 66 | 25 | 37.9 |
| 80 | 21 | 12 | 57.1 |
| Total | 153 | 48 | 39.2 |

| Normal control group, prevalence with VAF of 1.5 to 30% | | | |
|---|---|---|---|
| Ages | Number of samples | Positive (n) | Prevalence (%) |
| 50 | 2006 | 340 | 16.9 |
| 60 | 1003 | 260 | 25.9 |
| 70 | 257 | 93 | 36.2 |
| 80 | 12 | 3 | 25.0 |
| Total | 3278 | 696 | 21.2 |

Referring to Table 7, it was identified that the prevalence for the group of 89 genes was significantly higher in the patient group having wet-type age-related macular degeneration than the normal control group (39.2% vs 21.2%, p-value < 0.001). Among the group of 89 genes, genes showing a somatic variant with VAF of 1.5% or higher were identified. As a result, a total of 28 genes were selected as follows: APC, ASXL1, ASXL2, CD58, CHEK2, CUX1, DNMT3A, EP300, EZH2, GNB1, JAK1, JAK2, JARID2, KMT2D, NF1, NOTCH2, PPM1D, RIT1, SETD2, SF1, SF3B1, SRSF2, STAT3, SUZ12, TBL1XR1, TET2, TNFAIP3, and U2AF1. From the results, it can be seen that risk of wet-type age-related macular degeneration is significantly high in a case where a somatic variant exists in any one or more of the above genes.

### Example 3.2. Identification of prevalence for key genes

### Example 3.2.1. Prevalence analysis in patients having age-related macular degeneration

Prevalence for DNMT3A, TET2, and ASXL1 genes was checked in the patient group having age-related macular degeneration and the control group, and difference in the prevalence was analyzed by chi-square test. The analysis results are shown in Table 8.

**[Table 8]**

| Patient group having AMD, prevalence with VAF of 1.5 to 30% | | | |
|---|---|---|---|
| Ages | Number of samples | Positive (n) | Prevalence (%) |
| 50 | 15 | 2 | 13.3 |
| 60 | 69 | 16 | 23.2 |
| 70 | 92 | 24 | 26.1 |
| 80 | 21 | 9 | 42.9 |
| Total | 197 | 51 | 25.9 |

| Normal control group, prevalence with VAF of 1.5 to 30% | | | |
|---|---|---|---|
| Ages | Number of samples | Positive (n) | Prevalence (%) |
| 50 | 2006 | 173 | 8.6 |
| 60 | 1003 | 164 | 16.4 |
| 70 | 257 | 59 | 23.0 |
| 80 | 12 | 3 | 25.0 |
| Total | 3278 | 399 | 12.2 |

Referring to Table 8, as a result of the analysis, the prevalence of the patient group was significantly higher than that of the control group (25.9% vs 12.2%, p-value < 0.001).

### Example 3.2.2. Prevalence analysis in patients having wet-type age-related macular degeneration

The same analysis was performed on the 153 samples of wet-type age-related macular degeneration among the 197 samples from the entire patient group having age-related macular degeneration which were analyzed in Example 3.2.1., and the analysis results are shown in Table 9.

**[Table 9]**

| Patient group having AMD, prevalence with VAF of 1.5 to 30% | | | |
|---|---|---|---|
| Ages | Number of samples | Positive (n) | Prevalence (%) |
| 50 | 6 | 1 | 16.7 |
| 60 | 60 | 16 | 26.7 |
| 70 | 66 | 17 | 25.8 |
| 80 | 21 | 9 | 42.9 |
| Total | 153 | 43 | 28.1 |

| Normal control group, prevalence with VAF of 1.5 to 30% | | | |
|---|---|---|---|
| Ages | Number of samples | Positive (n) | Prevalence (%) |
| 50 | 2006 | 173 | 8.6 |
| 60 | 1003 | 164 | 16.4 |
| 70 | 257 | 59 | 23.0 |
| 80 | 12 | 3 | 25.0 |
| Total | 3278 | 399 | 12.2 |

Referring to Table 9, it was identified that the prevalence for the group of 89 genes was significantly higher in the patient group having wet-type age-related macular degeneration than in the normal control group (28.1% vs 12.2%, p-value < 0.001). In addition, logistic regression analysis was performed with adjustment of age, gender, and smoking status to examine effects of the three genes (DNMT3A, TET2, and ASXL1) on wet-type age-related macular degeneration. As a result, in a case where a somatic variant existed at an odds ratio of 1.57 (CI 1.02-2.40, p-value 0.0383) in the DNMT3A, TET2, or ASXL1 gene, there was significant association with wet-type age-related macular degeneration.

### Example 3.3. Identification of prevalence for individual genes

Prevalence for each of the individual genes (DNMT3A and TET2) was checked in the patient group and the control group, and difference in the prevalence was analyzed by chi-square test.

### Example 3.3.1. Prevalence analysis for TET2 in patients having age-related macular degeneration

First, the analysis results for the TET2 gene are shown in Table 10.

**[Table 10]**

| Patient group having AMD, prevalence with VAF of 1.5 to 30% | | | |
|---|---|---|---|
| Ages | Number of samples | Positive (n) | Prevalence (%) |
| 50 | 15 | 1 | 6.7 |
| 60 | 69 | 4 | 5.8 |
| 70 | 92 | 8 | 8.7 |
| 80 | 21 | 4 | 19.0 |
| Total | 197 | 17 | 8.6 |

| Normal control group, prevalence with VAF of 1.5 to 30% | | | |
|---|---|---|---|
| Ages | Number of samples | Positive (n) | Prevalence (%) |
| 50 | 2006 | 25 | 1.2 |
| 60 | 1003 | 40 | 4.0 |
| 70 | 257 | 13 | 5.1 |
| 80 | 12 | 0 | 0.0 |
| Total | 3278 | 78 | 2.4 |

Referring to Table 10, the prevalence of the TET2 gene was significantly higher in the patient group than in the control group (8.6% vs 2.4%, p-value < 0.001). In addition, an effect of the TET2 gene on age-related macular degeneration was examined by logistic regression analysis. The analysis was performed with adjustment of age, gender, and smoking status. As a result, it was identified that risk of macular degeneration significantly increased in a case where a somatic variant with a VAF of 1.5% or higher existed in the TET2 gene (OR 2.13, CI 1.11-4.11, p-value = 0.0235).

### Example 3.3.2. Prevalence analysis for TET2 in patients having wet-type age-related macular degeneration

The same analysis was performed on the 153 samples of wet-type age-related macular degeneration among all samples, and the analysis results are shown in Table 11.

**[Table 11]**

| Patient group having AMD, prevalence with VAF of 1.5 to 30% | | | |
|---|---|---|---|
| Ages | Number of samples | Positive (n) | Prevalence (%) |
| 50 | 6 | 0 | 0.0 |
| 60 | 60 | 4 | 6.7 |
| 70 | 66 | 6 | 9.1 |
| 80 | 21 | 4 | 19.0 |
| Total | 153 | 14 | 9.2 |

| Normal control group, prevalence with VAF of 1.5 to 30% | | | |
|---|---|---|---|
| Ages | Number of samples | Positive (n) | Prevalence (%) |
| 50 | 2006 | 25 | 1.2 |
| 60 | 1003 | 40 | 4.0 |
| 70 | 257 | 13 | 5.1 |
| 80 | 12 | 0 | 0.0 |
| Total | 3278 | 78 | 2.4 |

Referring to Table 11, the prevalence of the TET2 gene was significantly higher in the patient group than in the control group (9.2% vs 2.4%, p-value < 0.001). In addition, logistic regression analysis was performed with adjustment of age, gender, and smoking status to examine an effect of the TET2 gene on wet-type age-related macular degeneration. As a result, risk of wet age-related macular degeneration tended to increase in a case where a somatic variant existed at an odds ratio of 2.02 (CI 0.99-4.16, p-value 0.0548) in the TET2 gene.

### Example 3.3.3. Prevalence analysis of DNMT3A in patients having age-related macular degeneration

Next, the analysis results for the DNMT3A gene are shown in Table 12.

**[Table 12]**

| Patient group having AMD, prevalence with VAF of 1.5 to 30% | | | |
|---|---|---|---|
| Ages | Number of samples | Positive (n) | Prevalence (%) |
| 50 | 15 | 1 | 6.7 |
| 60 | 69 | 8 | 11.6 |
| 70 | 92 | 18 | 19.6 |
| 80 | 21 | 5 | 23.8 |
| Total | 197 | 32 | 16.2 |

| Normal control group, prevalence with VAF of 1.5 to 30% | | | |
|---|---|---|---|
| Ages | Number of samples | Positive (n) | Prevalence (%) |
| 50 | 2006 | 136 | 6.8 |
| 60 | 1003 | 111 | 11.1 |
| 70 | 257 | 40 | 15.6 |
| 80 | 12 | 3 | 25.0 |
| Total | 3278 | 290 | 8.8 |

Referring to Table 12, the prevalence of the DNMT3A gene was significantly higher in the patient group than in the control group (16.2% vs 8.8%, p-value < 0.001).

### Example 3.3.4. Prevalence analysis of DNMT3A in patients having wet-type age-related macular degeneration

The same analysis was performed on the 153 samples of wet-type age-related macular degeneration among all samples, and the analysis results are shown in Table 13.

**[Table 13]**

| Patient group having AMD, prevalence with VAF of 1.5 to 30% | | | |
|---|---|---|---|
| Ages | Number of samples | Positive (n) | Prevalence (%) |
| 50 | 6 | 1 | 16.7 |
| 60 | 60 | 8 | 13.3 |
| 70 | 66 | 14 | 21.2 |
| 80 | 21 | 5 | 23.8 |
| Total | 153 | 28 | 18.3 |

| Normal control group, prevalence with VAF of 1.5 to 30% | | | |
|---|---|---|---|
| Ages | Number of samples | Positive (n) | Prevalence (%) |
| 50 | 2006 | 136 | 6.8 |
| 60 | 1003 | 111 | 11.1 |
| 70 | 257 | 40 | 15.6 |
| 80 | 12 | 3 | 25.0 |
| Total | 3278 | 290 | 8.8 |

Referring to Table 13, the prevalence of the DNMT3A gene was significantly higher in the patient group than in the control group (18.3% vs 8.8%, p-value < 0.001).

### Example 4. Data analysis II

For the samples obtained in Example 1, a somatic variant was detected and screened in the same manner as in Example 2, except that a case where genetic variation with a VAF of 2.0% or higher is detected was defined as positive.

### Example 4.1. Identification of genes showing prevalence

### Example 4.1.1. Prevalence analysis in patients having age-related macular degeneration

For the 197 samples from the patient group having age-related macular degeneration and the 3278 samples from the normal control group, prevalence of 89 genes was checked, and difference in the prevalence was analyzed by chi-square test. Here, a subject was defined as positive in a case where a genetic variation with a variant allele fraction (VAF) of 2% or higher was detected in any one of the target genes. The analysis results are shown in Table 14.

**[Table 14]**

| Patient group having AMD, prevalence with VAF of 2 to 30% | | | |
|---|---|---|---|
| Ages | Number of samples | Positive (n) | Prevalence (%) |
| 50 | 15 | 2 | 13.3 |
| 60 | 69 | 15 | 21.7 |
| 70 | 92 | 30 | 32.6 |
| 80 | 21 | 11 | 52.4 |
| Total | 197 | 58 | 29.4 |

| Normal control group, prevalence with VAF of 2 to 30% | | | |
|---|---|---|---|
| Ages | Number of samples | Positive (n) | Prevalence (%) |
| 50 | 2006 | 236 | 11.8 |
| 60 | 1003 | 192 | 19.1 |
| 70 | 257 | 73 | 28.4 |
| 80 | 12 | 2 | 16.7 |
| Total | 3278 | 503 | 15.3 |

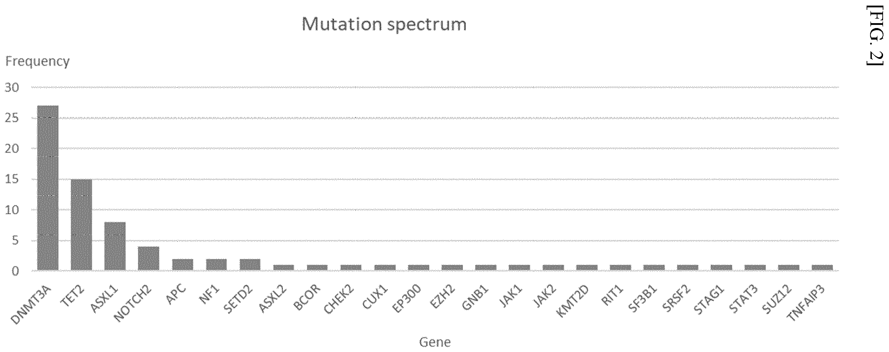

Referring to Table 14, it was identified that the prevalence for the group of 89 genes was significantly higher in the patient group having age-related macular degeneration than in the normal control group (29.4% vs 15.3%, p-value < 0.001). Among the group of 89 genes, genes showing a somatic variant with a VAF of 2.0% or higher were identified. As a result, a total of 24 genes were selected as follows: DNMT3A, TET2, ASXL1, APC, ASXL2, BCOR, CHEK2, CUX1, EP300, EZH2, GNB1, JAK1, JAK2, KMT2D, NF1, NOTCH2, RIT1, SETD2, SF3B1, SRSF2, STAG1, STAT3, SUZ12, and TNFAIP3. Detection frequency of each gene is illustrated in FIG. 2. From the results, it can be seen that risk of age-related macular degeneration is significantly high in a case where a somatic variant exists in any one or more of the above genes.

### Example 4.1.2. Prevalence analysis in patients having wet-type age-related macular degeneration

The same analysis was performed on the 153 samples of wet-type age-related macular degeneration among the 197 samples from the entire patient group having age-related macular degeneration which were analyzed in Example 4.1.1., and the analysis results are shown in Table 15.

**[Table 15]**

| Patient group having AMD, prevalence with VAF of 2 to 30% | | | |
|---|---|---|---|
| Ages | Number of samples | Positive (n) | Prevalence (%) |
| 50 | 6 | 1 | 16.7 |
| 60 | 60 | 15 | 25.0 |
| 70 | 66 | 21 | 31.8 |
| 80 | 21 | 11 | 52.4 |
| Total | 153 | 48 | 31.4 |

| Normal control group, prevalence with VAF of 2 to 30% | | | |
|---|---|---|---|
| Ages | Number of samples | Positive (n) | Prevalence (%) |
| 50 | 2006 | 236 | 11.8 |
| 60 | 1003 | 192 | 19.1 |
| 70 | 257 | 73 | 28.4 |
| 80 | 12 | 2 | 16.7 |
| Total | 3278 | 503 | 15.3 |

Referring to Table 15, it was identified that the prevalence for the group of 89 genes was significantly higher in the patient group having wet-type age-related macular degeneration than in the normal control group (31.4% vs 15.3%, p-value < 0.001). Among the group of 89 genes, genes showing a somatic variant with a VAF of 2.0% or higher were identified. As a result, a total of 21 genes were selected as follows: DNMT3A, TET2, ASXL1, SETD2, KMT2D, NF1, NOTCH2, SF3B1, ASXL2, CHEK2, CUX1, EZH2, GNB1, JAK1, JAK2, RIT1, SRSF2, SUZ12, APC, STAT3, and TNFAIP3. From the results, it can be seen that risk of wet-type age-related macular degeneration is significantly high in a case where a somatic variant exists in any one or more of the above genes.

### Example 4.2. Identification of prevalence for key genes

### Example 4.2.1. Prevalence analysis in patients having age-related macular degeneration

Prevalence for the three genes (DNMT3A, TET2, and ASXL1) was checked in the patient group having age-related macular degeneration and the control group, and difference in the prevalence was analyzed by chi-square test. The analysis results are shown in Table 16.

**[Table 16]**

| Patient group having AMD | | | |
|---|---|---|---|
| Ages | Number of samples | Positive (n) | Prevalence (%) |
| 50 | 15 | 2 | 13.3 |
| 60 | 69 | 9 | 13.0 |
| 70 | 92 | 21 | 22.8 |
| 80 | 21 | 8 | 38.1 |
| Total | 197 | 40 | 20.3 |

| Normal control group | | | |
|---|---|---|---|
| Ages | Number of samples | Positive (n) | Prevalence (%) |
| 50 | 2006 | 132 | 6.6 |
| 60 | 1003 | 130 | 13.3 |
| 70 | 257 | 45 | 17.5 |
| 80 | 12 | 2 | 16.7 |
| Total | 3278 | 309 | 9.4 |

Referring to Table 16, as a result of the analysis, the prevalence of the patient group was significantly higher than that of the control group (20.3% vs 9.4%, p-value < 0.001).

### Example 4.2.2. Prevalence analysis in patients having wet-type age-related macular degeneration

The same analysis was performed on the 153 samples of wet-type age-related macular degeneration among the 197 samples from the entire patient group having age-related macular degeneration which were analyzed in Example 4.2.1., and the analysis results are shown in Table 17.

**[Table 17]**

| Patient group having AMD, prevalence with VAF of 2 to 30% | | | |
|---|---|---|---|
| Ages | Number of samples | Positive (n) | Prevalence (%) |
| 50 | 6 | 1 | 16.7 |
| 60 | 60 | 9 | 15.0 |
| 70 | 66 | 15 | 22.7 |
| 80 | 21 | 8 | 38.1 |
| Total | 153 | 33 | 21.6 |

| Normal control group, prevalence with VAF of 2 to 30% | | | |
|---|---|---|---|
| Ages | Number of samples | Positive (n) | Prevalence (%) |
| 50 | 2006 | 132 | 6.6 |
| 60 | 1003 | 130 | 13.3 |
| 70 | 257 | 45 | 17.5 |
| 80 | 12 | 2 | 16.7 |
| Total | 3278 | 309 | 9.4 |

Referring to Table 17, it was identified that the prevalence for the group of 89 genes was significantly higher in the patient group having wet-type age-related macular degeneration than in the normal control group (21.6% vs 9.4%, p-value < 0.001). In addition, logistic regression analysis was performed with adjustment of age, gender, and smoking status to examine effects of the three genes (DNMT3A, TET2, and ASXL1) on wet-type age-related macular degeneration. As a result, in a case where a somatic variant existed at an odds ratio of 1.37 (CI 0.86-2.20, p-value 0.1881) in the DNMT3A, TET2, or ASXL1 gene, there was significant association with wet-type age-related macular degeneration.

### Example 4.3. Identification of prevalence for individual genes

Prevalence for each of the individual genes (DNMT3A and TET2) was checked in the patient group and the control group, and difference in the prevalence was analyzed by chi-square test.

### Example 4.3.1. Prevalence analysis for TET2 in patients having age-related macular degeneration

First, the analysis results for the TET2 gene are shown in Table 18.

**[Table 18]**

| Patient group having AMD, prevalence with VAF of 2 to 30% | | | |
|---|---|---|---|
| Ages | Number of samples | Positive (n) | Prevalence (%) |
| 50 | 15 | 1 | 6.7 |
| 60 | 69 | 2 | 2.9 |
| 70 | 92 | 6 | 6.5 |
| 80 | 21 | 4 | 19.0 |
| Total | 197 | 13 | 6.6 |

| Normal control group, prevalence with VAF of 2 to 30% | | | |
|---|---|---|---|
| Ages | Number of samples | Positive (n) | Prevalence (%) |
| 50 | 2006 | 19 | 0.9 |
| 60 | 1003 | 29 | 2.9 |
| 70 | 257 | 10 | 3.9 |
| 80 | 12 | 0 | 0.0 |
| Total | 3278 | 58 | 1.8 |

Referring to Table 18, the prevalence of the TET2 gene was significantly higher in the patient group than in the control group (6.6% vs 1.8%, p-value < 0.001). In addition, an effect of the TET2 gene on age-related macular degeneration was examined by logistic regression analysis. The analysis was performed with adjustment of age, gender, and smoking status. As a result, it was identified that risk of macular degeneration tends to increase in a case where a somatic variant existed in the TET2 gene (OR 1.93, CI 0.90-4.16, p-value = 0.0913).

### Example 4.3.2. Prevalence analysis for TET2 in patients having wet-type age-related macular degeneration

The same analysis was performed on the 153 samples of wet-type age-related macular degeneration among all samples, and the analysis results are shown in Table 19.

**[Table 19]**

| Patient group having AMD, prevalence with VAF of 2 to 30% | | | |
|---|---|---|---|
| Ages | Number of samples | Positive (n) | Prevalence (%) |
| 50 | 6 | 0 | 0.00 |
| 60 | 60 | 2 | 3.3 |
| 70 | 66 | 5 | 7.6 |
| 80 | 21 | 4 | 19.0 |
| Total | 153 | 11 | 7.2 |

| Normal control group, prevalence with VAF of 2 to 30% | | | |
|---|---|---|---|
| Ages | Number of samples | Positive (n) | Prevalence (%) |
| 50 | 2006 | 19 | 0.9 |
| 60 | 1003 | 29 | 2.9 |
| 70 | 257 | 10 | 3.9 |
| 80 | 12 | 0 | 0.0 |
| Total | 3278 | 58 | 1.8 |

Referring to Table 19, the prevalence of the TET2 gene was significantly higher in the patient group than in the control group (7.2% vs 1.8%, p-value < 0.001). In addition, logistic regression analysis was performed with adjustment of age, gender, and smoking status to examine an effect of the TET2 gene on wet-type age-related macular degeneration. As a result, risk of wet-type macular degeneration tends to increase in a case where a somatic variant existed at an odds ratio of 1.83 (CI 0.80-4.20, p-value 0.1519) in the TET2 gene.

### Example 4.3.3. Prevalence analysis for DNMT3A in patients having age-related macular degeneration

Next, the analysis results for the gene are shown in Table 20.

**[Table 20]**

| Patient group having AMD | | | |
|---|---|---|---|
| Ages | Number of samples | Positive (n) | Prevalence (%) |
| 50 | 15 | 1 | 6.7 |
| 60 | 69 | 3 | 4.3 |
| 70 | 92 | 15 | 16.3 |
| 80 | 21 | 4 | 19.0 |
| Total | 197 | 23 | 11.7 |

| Normal control group | | | |
|---|---|---|---|
| Ages | Number of samples | Positive (n) | Prevalence (%) |
| 50 | 2006 | 105 | 5.2 |
| 60 | 1003 | 87 | 8.7 |
| 70 | 257 | 32 | 12.5 |
| 80 | 12 | 2 | 16.7 |
| Total | 3278 | 226 | 6.9 |

Referring to Table 20, the prevalence of the DNMT3A gene was significantly higher in the patient group than in the control group (11.7% vs 6.9%, p-value = 0.011).

### Example 4.3.4. Prevalence analysis for DNMT3A in patients having wet-type age-related macular degeneration

The same analysis was performed on 77 samples of wet-type age-related macular degeneration among all samples, and the analysis results are shown in Table 21.

**[Table 21]**

| Patient group having AMD, prevalence with VAF of 2 to 30% | | | |
|---|---|---|---|
| Ages | Number of samples | Positive (n) | Prevalence (%) |
| 50 | 6 | 1 | 16.7 |
| 60 | 60 | 3 | 5.0 |
| 70 | 66 | 11 | 16.7 |
| 80 | 21 | 4 | 19.0 |
| Total | 153 | 19 | 12.4 |

| Normal control group, prevalence with VAF of 2 to 30% | | | |
|---|---|---|---|
| Ages | Number of samples | Positive (n) | Prevalence (%) |
| 50 | 2006 | 105 | 5.2 |
| 60 | 1003 | 87 | 8.7 |
| 70 | 257 | 32 | 12.5 |
| 80 | 12 | 2 | 16.7 |
| Total | 3278 | 226 | 6.9 |

Referring to Table 21, the prevalence of the DNMT3A gene was significantly higher in the patient group than in the control group (12.4% vs 6.9%, p-value = 0.009).

## Claims

1. A composition for providing information necessary for predicting occurrence of, diagnosing, or treating age-related macular degeneration, the composition comprising an agent(s) capable of detecting a clonal hematopoiesis-inducing mutation(s) using a biological sample isolated from a subject.

2. The composition of claim 1, wherein the mutation(s) comprises mutation(s) in one or more genes selected from the group consisting of APC, ASXL1, ASXL2, BCOR, CD58, CHEK2, CUX1, DNMT3A, EP300, EZH2, GNB1, JAK1, JAK2, JARID2, KMT2D, NF1, NOTCH2, PPM1D, RIT1, SETD2, SF1, SF3B1, SRSF2, STAG1, STAT3, SUZ12, TBL1XR1, TET2, TNFAIP3, and U2AF1.

3. The composition of claim 1, wherein the mutation(s) comprises a mutation(s) in one or more genes selected from the group consisting of APC, ASXL1, ASXL2, CD58, CHEK2, CUX1, DNMT3A, EP300, EZH2, GNB1, JAK1, JAK2, JARID2, KMT2D, NF1, NOTCH2, PPM1D, RIT1, SETD2, SF1, SF3B1, SRSF2, STAT3, SUZ12, TBL1XR1, TET2, TNFAIP3, and U2AF1.

4. The composition of claim 1, wherein the mutation(s) comprises a mutation(s) in one or more genes selected from the group consisting of DNMT3A, TET2, ASXL1, APC, ASXL2, BCOR, CHEK2, CUX1, EP300, EZH2, GNB1, JAK1, JAK2, KMT2D, NF1, NOTCH2, RIT1, SETD2, SF3B1, SRSF2, STAG1, STAT3, SUZ12, and TNFAIP3.

5. The composition of claim 1, wherein the mutation(s) comprises a mutation(s) in one or more genes selected from the group consisting of DNMT3A, TET2, ASXL1, SETD2, KMT2D, NF1, NOTCH2, SF3B1, ASXL2, CHEK2, CUX1, EZH2, GNB1, JAK1, JAK2, RIT1, SRSF2, SUZ12, APC, STAT3, and TNFAIP3.

6. The composition of claim 1, wherein the mutation(s) comprises a mutation(s) in one or more genes selected from the group consisting of DNMT3A, TET2, and ASXL1.

7. The composition of claim 1, wherein the mutation(s) is a missense mutation, a frameshift mutation, a nonsense mutation, or a splice mutation.

8. The composition of claim 1, wherein the agent(s) includes a primer, a probe, or antisense nucleic acid for detecting the mutation(s).

9. The composition of claim 1, wherein the age-related macular degeneration is wet-type macular degeneration.

10. A kit for providing information necessary for predicting occurrence of, diagnosing, or treating age-related macular degeneration, the kit comprising the composition of any one of claims 1 to 9.

11. A genetic analysis panel for providing information necessary for predicting occurrence of, diagnosing, or treating age-related macular degeneration, the panel comprising the composition of any one of claims 1 to 9.

12. A method for providing information for predicting occurrence of, diagnosing, or treating age-related macular degeneration, the method comprising determining whether a clonal hematopoiesis-inducing mutation(s) exists in a subject through genetic analysis of a biological sample isolated from the subject.

13. The method of claim 12, wherein the mutation(s) comprises a mutation(s) in one or more genes selected from the group consisting of APC, ASXL1, ASXL2, BCOR, CD58, CHEK2, CUX1, DNMT3A, EP300, EZH2, GNB1, JAK1, JAK2, JARID2, KMT2D, NF1, NOTCH2, PPM1D, RIT1, SETD2, SF1, SF3B1, SRSF2, STAG1, STAT3, SUZ12, TBL1XR1, TET2, TNFAIP3, and U2AF1.

14. The method of claim 12, wherein the mutation(s) comprises a mutation(s) in one or more genes selected from the group consisting of APC, ASXL1, ASXL2, CD58, CHEK2, CUX1, DNMT3A, EP300, EZH2, GNB1, JAK1, JAK2, JARID2, KMT2D, NF1, NOTCH2, PPM1D, RIT1, SETD2, SF1, SF3B1, SRSF2, STAT3, SUZ12, TBL1XR1, TET2, TNFAIP3, and U2AF 1.

15. The method of claim 12, wherein the mutation(s) comprises a mutation(s) in one or more genes selected from the group consisting of DNMT3A, TET2, ASXL1, APC, ASXL2, BCOR, CHEK2, CUX1, EP300, EZH2, GNB1, JAK1, JAK2, KMT2D, NF1, NOTCH2, RIT1, SETD2, SF3B1, SRSF2, STAG1, STAT3, SUZ12, and TNFAIP3.

16. The method of claim 12, wherein the mutation(s) comprises a mutation(s) in one or more genes selected from the group consisting of DNMT3A, TET2, ASXL1, SETD2, KMT2D, NF1, NOTCH2, SF3B1, ASXL2, CHEK2, CUX1, EZH2, GNB1, JAK1, JAK2, RIT1, SRSF2, SUZ12, APC, STAT3, and TNFAIP3.

17. The method of claim 12, wherein the mutation(s) comprises a mutation(s) in one or more genes selected from the group consisting of DNMT3A, TET2, and ASXL1.

18. The method of claim 12, wherein the mutation(s) is a missense mutation, a frameshift mutation, a nonsense mutation, or a splice mutation.

19. The method of claim 12, wherein the biological sample is blood, serum, plasma, lymph fluid, saliva, sputum, mucus, urine, or feces.

20. The method of claim 12, wherein the genetic analysis is performed using next generation sequencing.

21. The method of claim 12, further comprising:
determining that age-related macular degeneration is highly likely to occur in a case where the mutation(s) exists.

22. The method of claim 12, further comprising:
determining that in a case where the mutation(s) exists, it is necessary to perform a treatment in the subject so that the mutation(s) is suppressed or a function of the gene, in which the mutation(s) exists, is restored or supplemented, in order to decrease risk of occurrence or progression of age-related macular degeneration.

23. The method of claim 12, wherein the age-related macular degeneration is wet-type macular degeneration.
